# FASCICULE DE BREVET EUROPEEN

(11) **EP 3 026 647 B1**
(45) Date de publication et mention de la délivrance du brevet: **31.08.2022**
(21) Numéro de dépôt: 15196550.6
(22) Date de dépôt: 26.11.2015
(51) Int. Cl.: G08B 21/04

(54) **PROCÉDÉ DE QUALIFICATION D'ALERTE PARMI DES ALERTES ISSUES D'UN SYSTÈME DE SUPERVISION D'ACTIVITÉ**
VERFAHREN ZUR QUALIFIZIERUNG VON WARNMELDUNGEN, DIE VON EINEM BETRIEBSÜBERWACHUNGSSYSTEM AUSGEGEBEN WERDEN
METHOD FOR QUALIFYING AN ALERT AMONG ALERTS FROM AN ACTIVITY SUPERVISION SYSTEM

(30) Priorité: 28.11.2014 FR 1461649
(43) Date de publication de la demande: 01.06.2016
(73) Titulaire: Orange, 92130 Issy-les-Moulineaux (FR)
(72) Inventeur: BERENGUER, Marc, 38420 Revel (FR); BOUZID, Marie-Jeanne, Echirolles (FR)

(56) Documents cités:
- EP-A2- 2 472 487
- US-A1- 2008 001 735
- NOURY N ET AL: "Use of electrical devices reveals our well being", ENGINEERING IN MEDICINE AND BIOLOGY SOCIETY,EMBC, 2011 ANNUAL INTERNATIONAL CONFERENCE OF THE IEEE, IEEE, 30 août 2011 (2011-08-30), pages 1769-1772, XP032319078, DOI: 10.1109/IEMBS.2011.6090505 ISBN: 978-1-4244-4121-1

## Description

### DOMAINE TECHNIQUE

La présente invention se rapporte au domaine de la télésurveillance médicale et concerne particulièrement un système d'aide au maintien à domicile de personnes âgées, dépendantes ou fragiles.

### ART ANTERIEUR

L'invention trouve une application particulièrement avantageuse dans le suivi de personnes âgées vivant seules à domicile, mais peut s'appliquer à d'autres systèmes de suivi d'activité.

Le vieillissement de la population dans les pays développés couplée à un manque de structures pour recevoir les personnes âgées, implique de maintenir ces populations à domicile le plus longtemps possible et dans les meilleures conditions.

On connait des systèmes non intrusifs permettant de suivre l'activité de personnes vivant seules à domicile. En particulier, la publication intitulée « Use of Electrical Devices Reveals Our Well Being » présentée lors de la conférence annuelle EMBC (Engineering in Medicine and Biology Society, 2011, IEEE, N. Noury, K-A Quach, M. Berenguer, H.Teyssier, M-J Bouzid, L. Goldstein, M. Giordani) décrit un système de suivi d'activité basé sur l'utilisation des appareils électriques du domicile. Le système n'utilise qu'un seul capteur apte à identifier le déclenchement des différents dispositifs électriques au domicile de la personne suivie et permet de mettre en relation directe des données électriques avec l'activité de la personne.

De tels systèmes analysent l'activité des personnes pendant une première période d'initialisation au cours de laquelle différents indicateurs sont mesurés et mémorisés. Les indicateurs peuvent par exemple se rapporter à des données relatives à l'utilisation d'appareils électriques, des données relatives aux communications téléphoniques ou encore à des données physiologiques comme une pesée par exemple. Les périodes d'initialisation peuvent être de durées différentes suivant les indicateurs considérés. Une analyse statistique de ces données d'apprentissage permet de créer un référentiel spécifique à une situation particulière définie par les caractéristiques de l'environnement, l'âge, le sexe ou les antécédents de la personne supervisée.

Suite à cette période d'initialisation, le système de suivi est opérationnel et les indicateurs considérés sont mesurés périodiquement, par exemple toutes les 24 heures. À partir de ces données, un indice d'activité correspondant à la somme des écarts observés entre les indicateurs du référentiel et les indicateurs périodiques est établit. Lorsqu'une dérive importante de cet indice d'activité est constatée, une alerte est déclenchée afin par exemple d'attirer l'attention de la famille ou d'un plateau d'assistance sur une modification sensible du comportement de la personne suivie. Ainsi, il est par exemple possible de détecter un changement de comportement nocturne qu'une visite ponctuelle n'aurait pu déceler.

Toutefois, malgré tout l'intérêt que présente un tel système, il présente l'inconvénient de déclencher de fausses alertes dans certains cas. Ces fausses alertes sont indésirables car elles mobilisent inutilement des ressources techniques et humaines et peuvent être source d'inquiétude pour les proches.

Considérons par exemple le cas d'une personne suivie qui a l'habitude de rejoindre son lit vers 22h. Une sonde connectée au réseau électrique renseigne quotidiennement un indicateur relatif à l'éclairage de l'habitation. Cet indicateur permet généralement d'établir que la personne est bien dans sa chambre à 22h. En confrontant l'indicateur aux données mémorisées lors de la période d'initialisation, le système vérifie que l'habitude est bien observée et ne signale pas d'alerte particulière. En revanche, si la personne commence à être sujette à des insomnies et se couche beaucoup plus tard, la confrontation des indicateurs avec les données mémorisées lors de la période d'initialisation permettront de déclencher une alerte. Or, lorsque par exemple ce couchage tardif est dû à une compétition sportive internationale diffusée en direct à la télévision, le déclenchement de l'alerte est inapproprié. De nombreux autres facteurs, tels que par exemple les changements d'heure ou de saison, peuvent être la source de fausses alertes.

Une solution peut consister à réinitialiser le système lorsque des changements importants ont lieu, par exemple en début de saison, mais le suivi d'activité est alors moins fiable pendant la nouvelle période d'initialisation. Une autre solution peut consister à programmer les évènements prévisibles, comme les changements d'heure, de façon à ce que le décalage soit pris en compte lors de la comparaison des indicateurs. Malgré tout, certains facteurs comme le changement d'heure ont un effet physiologique sur la personne qui n'est pas prévisible et le fait qu'un facteur externe soit connu à l'avance ne permettra pas toujours d'éviter le déclenchement d'une alerte.

On connait également des systèmes tels que celui décrit dans la demande de brevet EP 2 472 787 A2. Un tel système propose d'effectuer une comparaison d'une dérive d'un indice d'activité d'un système supervisé avec la dérive d'un indice similaire obtenu à partir d'un deuxième système.

Le document US 2008/0001735 A1 décrit un système de supervision mettant en œuvre des capteurs permettant de mesurer l'évolution de l'activité d'une personne afin d'en déduire une activité anormale.

Toutefois, les systèmes décrits dans ces documents sont susceptibles de lever de fausses alertes.

Il existe donc un besoin pour une solution technique permettant d'améliorer la fiabilité d'un système de suivi à domicile en limitant le taux de fausses alertes, notamment lorsqu'elles sont liées à des facteurs extérieurs à la situation.

### RÉSUMÉ DE L'INVENTION

À cet effet, l'invention concerne un procédé de qualification d'une alerte selon la revendication 1.

La dérive de l'indice correspond à l'écart constaté entre la valeur de cet indice à un instant donné et sa valeur telle qu'observée lors d'une période de référence. Par exemple, une période d'initialisation peut être observée suite à l'installation d'un système de supervision au domicile d'un individu. L'activité est mesurée pendant cette période à l'aide de capteurs afin d'établir un indice d'activité de l'individu. Les données collectées durant cette période d'initialisation servent de références auxquelles sont comparées des données mesurées quotidiennement à l'aide des mêmes capteurs de façon à détecter et quantifier une éventuelle dérive.

Lorsqu'une pluralité de systèmes de supervision est interconnectée, par exemple par l'intermédiaire d'un réseau de télécommunication et d'un serveur de gestion, le procédé permet de comparer l'évolution d'un indice d'activité d'un individu avec l'évolution d'un indice d'activité relatif à un ou d'autres individus. Il est ainsi possible de déterminer par exemple qu'un changement de comportement chez un individu correspond au même changement chez un autre individu. En effet, si un facteur externe tel qu'un changement d'heure ou une compétition sportive provoquent un changement de comportement chez un individu suivi, un tel changement de comportement sera probablement constaté chez d'autres individus et la situation pourra être reconsidérée comme étant normale, évitant ainsi le déclenchement d'une alerte inappropriée.

La qualification d'une alerte consiste ici à en évaluer la pertinence et d'y associer une donnée représentative de cette pertinence. Par exemple, la qualification d'une alerte peut consister à lui associer un état « confirmé » ou « infirmé », ou encore un degré de pertinence sur une échelle prédéterminée. Un faible degré de pertinence pour une alerte peut ainsi conduire à son annulation.

Selon un mode particulier de réalisation, le procédé est tel que l'étape de deuxième comparaison est réalisée à partir de la dérive de l'indicateur majoritaire du premier système de supervision et d'une moyenne de la dérive de l'indicateur majoritaire de la pluralité de deuxième systèmes.

La dérive constatée par le premier système de supervision est comparée avec la moyenne des dérives constatées par les autres systèmes de supervision reliés au serveur de gestion. Ainsi, le procédé peut qualifier une alerte de manière plus fine, en vérifiant que la dérive constatée par le premier système de supervision suit ou non la tendance moyenne des autres entités.

Selon une réalisation particulière, le procédé est tel que les systèmes de supervision sont associés à des contextes domestiques et en ce que les systèmes de supervision sont regroupés en classes définies par au moins un critère de similitude des contextes domestiques auxquels ils sont associés, l'étape de première comparaison étant réalisée à partir de la dérive de l'indice d'activité du premier système de supervision et d'une moyenne de la dérive de l'indice d'activité des systèmes de supervision appartenant à la même classe que le premier système.

Le contexte domestique se rapporte à l'environnement dans lequel est installé le système, comme par exemple la configuration d'un logement, le nombre de pièces ou d'étages, ou encore la localisation géographique de ce logement. Le contexte domestique peut également se rapporter à certaines caractéristiques de l'individu dont l'activité est suivie. Le procédé propose de regrouper des systèmes de supervision en classes selon des similitudes du contexte domestique. Par exemple, une classe peut correspondre à des personnes âgées vivant dans un appartement alors qu'une autre correspond à des personnes de la même tranche d'âge vivant dans une maison. Toute sorte de critères relatifs à l'environnement et à la physiologie de l'individu suivi peut être utilisée pour former des classes. De cette façon, le procédé permet de comparer la dérive de l'indice d'activité d'un individu relevé par un système de supervision avec une dérive moyenne relevée par les systèmes de supervision appartenant à la même classe. Les alertes émises suite à une dérive de l'indice d'activité peuvent ainsi être qualifiées de manière beaucoup plus pertinente.

Selon un mode particulier de réalisation, le procédé est tel que les dérives d'indice d'activité sont pondérées selon une date de mise en service du système de supervision correspondant.

Lorsque le système de supervision est un système expert fonctionnant par apprentissage, la fiabilité des indices mesurés augmente dans le temps. La pondération des dérives constatées sur les différents systèmes reliés au serveur de gestion en fonction de la date de mise en service du système permet donc de privilégier les données les plus fiables lors du calcul de la dérive moyenne de l'indice.

Selon l'invention, le procédé est tel que l'indice d'activité pour un système de supervision est établi à partir d'une pluralité d'indicateurs et qu'il comporte en outre les étapes suivantes :
- Identification de l'indicateur majoritaire ayant provoqué la dérive de l'indice du premier système,
- Obtention de la dérive de l'indicateur pour le au moins un deuxième système de supervision,
- Comparaison de la dérive de l'indicateur majoritaire du premier système de supervision et d'une valeur représentative de la dérive de l'indicateur du au moins un deuxième système, et
- Mise à jour de la qualification de l'alerte en fonction du résultat de la comparaison.

L'indice d'activité est mesuré à partir de différents indicateurs. Ces indicateurs peuvent par exemple être établis à partir de détecteurs de mouvements, de capteurs d'activité électrique ou d'un dispositif portable et rendre compte des activités quotidiennes de la personne suivie (hygiène, alimentation, télévision, ...). L'indice peut être calculé de manière à ce que lorsqu'un ou plusieurs indicateurs dérivent de leur valeur habituelle, il provoque une dérive de l'indice. L'observation des variations de l'indice permet ainsi de constater des variations du comportement habituel d'un individu. Toutefois, la seule comparaison de la dérive d'un tel indice constatée pour une installation avec une dérive moyenne du même indice constatée pour un ensemble d'installation ne suffit pas toujours à confirmer une tendance globale. Ainsi, le fait de comparer la dérive de l'indicateur principalement responsable de la dérive de l'indice avec la dérive moyenne de ce même indicateur dans d'autres installation permet ou non de confirmer la tendance, permettant ainsi de mettre à jour la qualification de l'alerte avec une meilleur précision. Selon une réalisation particulière, la valeur représentative de la dérive de l'indicateur du au moins un deuxième système correspond à la moyenne des écarts constatés pour cet indicateur par rapport à une valeur de référence, la moyenne pouvant être calculée uniquement pour les systèmes de supervision de même classe.

Selon une autre réalisation particulière, le procédé est tel que le au moins un critère de similitude entre les contextes domestiques comporte au moins un élément sélectionné parmi les éléments suivants :
- Une localisation géographique,
- La configuration spatiale d'un environnement, et
- Une caractéristique physiologique de l'individu.

Les différents systèmes de supervision reliés au serveur de gestion sont regroupés en classes selon la similitude de leur contexte domestique. Le contexte domestique correspond à la situation supervisée et se rapporte à l'environnement domestique et à des caractéristiques physiologiques de la personne suivie. Par exemple, le contexte domestique comprend des caractéristiques du logement supervisé et de l'individu suivi. La comparaison de l'indice d'activité établi par un système de supervision avec l'indice établi par d'autres systèmes de supervision est d'autant plus pertinente que le contexte domestique des systèmes comparés est similaire.

En particulier, le procédé permet de mettre en correspondance des systèmes de supervision selon la localisation géographique de l'installation. La localisation géographique peut être par exemple un identifiant de commune, une rue ou encore une zone du territoire définie par des coordonnées GPS. Le critère de localisation géographique peut également se rapporter au fait qu'un système de supervision est installé en milieu urbain ou rural par exemple. Le procédé propose également de regrouper les systèmes de supervision selon la configuration spatiale d'un environnement. Par exemple, le procédé peut prendre en compte le fait qu'un système de supervision soit installé dans un logement de type appartement ou maison et prendre en considération le nombre de pièces de ce logement ainsi que par exemple le nombre d'étages. Le procédé prend également en compte des caractéristiques physiologiques d'un individu suivi pour établir des regroupements entre différents systèmes de supervisions. Par exemple, l'âge de la personne suivie, son poids, sa taille ou encore son sexe peuvent être pris en compte. La prise en compte de ces critères de similitude afin de répartir les systèmes de supervisions connectés au serveur de gestion dans des classes permet de dégager des tendances particulièrement pertinentes au regard du système considéré et d'améliorer nettement la fiabilité des alertes pouvant être déclenchées.

Une telle disposition présente en outre l'avantage de pouvoir prédire la survenue d'événements chez un individu lorsque des événements se sont déjà produits chez des individus ayant des contextes domestiques similaires. Ces prédictions peuvent être intéressantes pour des sociétés d'assurance ou d'aide à la personne par exemple.

Selon un autre aspect, l'invention concerne un dispositif de qualification tel que défini dans la revendication 6.

Selon encore un autre aspect non couvert par l'invention, est mentionné un procédé de demande de qualification d'une alerte sur un système de supervision supervisant l'activité d'un individu, le système étant adapté pour calculer un indice d'activité représentatif de l'activité de individu, des alertes étant déclenchées par le système de supervision lors d'une dérive de l'indice d'activité, une dérive correspondant à un écart de la valeur de l'indice par rapport à une valeur de référence, le procédé étant caractérisé en ce qu'il comporte les étapes suivantes suite au déclenchement (400) d'une alerte:
- Émission d'une requête de qualification d'alerte comportant une donnée relative à la dérive de l'indice d'activité,
- Réception d'un message comportant une qualification de l'alerte en fonction d'une comparaison entre la donnée relative à la dérive de l'indice d'activité du système de supervision et d'une donnée relative à la dérive de l'indice d'activité d'au moins un deuxième système de supervision, et
- Émission d'une alerte vers un serveur de traitement des alertes que lorsque l'alerte est qualifiée confirmée.

De cette façon, un système de supervision mettant en œuvre le procédé de demande de qualification peut consulter un serveur pour qualifier une alerte avant de solliciter une intervention au domicile de la personne suivie. Le système peut ainsi éviter le déclenchement d'une intervention inappropriée lorsqu'un changement de comportement s'inscrit dans une tendance globale.

L'invention concerne aussi un dispositif de demande de qualification d'une alerte selon la revendication 6.

L'invention concerne également un serveur comprenant un dispositif de qualification d'une alerte. L'invention se rapporte aussi un terminal comprenant un dispositif de demande de qualification d'une alerte.

L'invention concerne également un programme d'ordinateur comportant les instructions pour l'exécution du procédé de qualification et/ou les instructions pour l'exécution du procédé de demande de qualification, lorsque le programme est exécuté par un processeur.

L'invention concerne aussi un support d'informations lisible par un processeur sur lequel est enregistré un programme d'ordinateur comprenant des instructions pour l'exécution des étapes du procédé de qualification et/ou les étapes du procédé de demande de qualification.

Les différents modes ou caractéristiques de réalisation précités peuvent être ajoutés indépendamment ou en combinaison les uns avec les autres, aux étapes du procédé de qualification et/ou du procédé de demande de qualification tels que définis ci-dessus.

Les serveurs, terminaux, dispositifs, programmes et supports d'information présentent au moins des avantages analogues à ceux conférés par le procédé de composition décrit ci-dessus.

### LISTE DES FIGURES

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- La figure 1 représente une architecture réseau simplifiée adaptée pour la mise en œuvre de l'invention selon un mode de réalisation particulier,
- La figure 2 illustre les principales étapes du procédé de qualification selon une réalisation particulière,
- La figure 3 illustre les principales étapes du procédé de qualification selon une autre réalisation particulière,
- La figure 4 illustre les principales étapes du procédé de téléchargement selon un mode de réalisation particulier, et
- La figure 5 représente un schéma simplifié d'un dispositif adapté pour la mise en œuvre du procédé de qualification d'alerte selon un mode particulier de réalisation.
- La figure 6 représente un schéma simplifié d'un dispositif adapté pour la mise en œuvre du procédé de demande de qualification selon un mode particulier de réalisation.

### DESCRIPTION DÉTAILLÉE

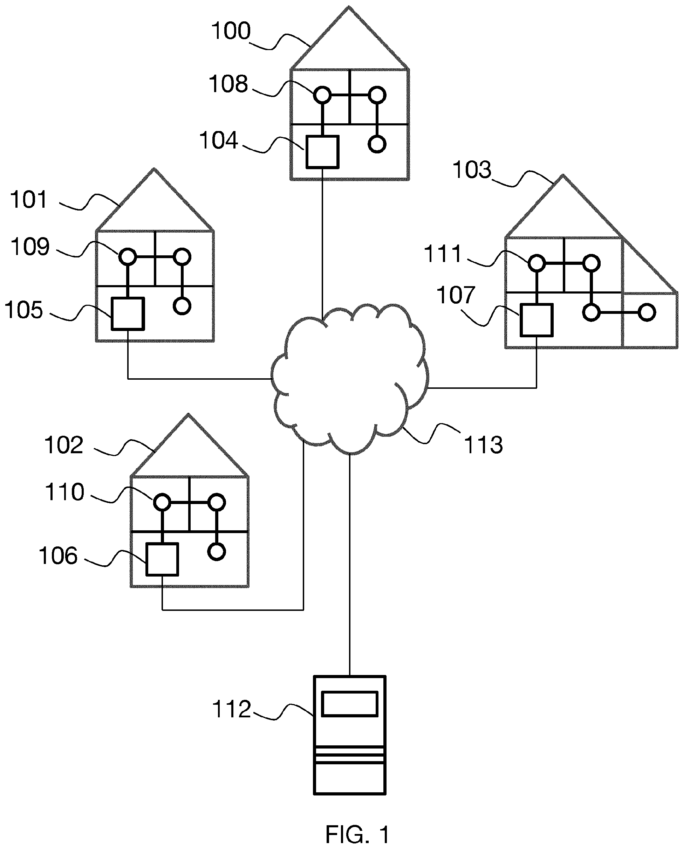

La **figure 1** représente une architecture réseau simplifiée adaptée pour la mise en œuvre de l'invention selon un mode de réalisation particulier, dans laquelle 4 habitations **100, 101, 102** et **103** sont équipées d'un système de supervision pour le suivi de l'activité d'un habitant. Ces 4 habitations sont respectivement équipées d'un dispositif de contrôle **104, 105, 106** et **107** et d'un réseau de capteurs **108, 109, 110** et **111.** Les 4 habitations sont en outre reliées à un serveur de gestion **112** au travers d'un réseau de communication **113.**

Le fonctionnement du système de supervision va maintenant être décrit en référence à l'habitation **100** de la figure 1. Les systèmes installés dans les autres habitations de la figure 1 présentent un fonctionnement identique. Selon une réalisation particulière, le système de supervision permet de suivre l'activité d'une personne dans l'habitation. Il peut s'agir par exemple d'un système tel que celui décrit dans le document « *Use of Electrical Devices Reveals Our Well Being »* cité en première partie de cet exposé. Un tel système utilise un détecteur d'activité électrique connecté au réseau électrique de l'habitation afin de tracer l'utilisation des différents appareils électriques de l'environnement et en déduire l'activité de la personne.

À partir de ces données d'utilisation, le système de supervision renseigne un certain nombre d'indicateurs d'activité de la vie courante de la personne. Par exemple, l'utilisation par un habitant d'un grille-pain ou d'une cafetière le matin permet de renseigner un indicateur relatif au fait que la personne prend un petit déjeuner à une certaine heure le matin. Toujours par exemple, la détection de l'utilisation d'appareils tels qu'un sèche-cheveux ou un rasoir permet de renseigner un indicateur relatif à l'hygiène de la personne.

Suite à l'installation du système de supervision dans l'habitation **100,** une période d'initialisation est engagée pendant laquelle l'activité de la personne est mémorisée dans une base de données, par exemple dans une base de données du dispositif de contrôle **104.** Les données recueillies peuvent également être mémorisées sur un serveur distant. Durant cette période, dite de référence, les habitudes et comportements de la personne suivie sont donc mémorisées sous la forme d'indicateurs.

À l'issue de la période d'initialisation, les indicateurs sont renseignés quotidiennement. La valeur de chacun des indicateurs étant comparée à la valeur de ces mêmes indicateurs telle que mémorisée durant la période de référence. Une attention particulière est portée à l'écart de ces indicateurs par rapport à la valeur de référence mémorisée pendant la phase d'initialisation. Un indice correspondant à la somme des écarts mesurés pour les différents indicateurs est ainsi calculé quotidiennement. Cet indice d'activité permet de détecter des changements dans le comportement de la personne suivie. Lorsque l'indice dérive de façon importante par rapport à sa valeur de référence calculée pendant la période d'initialisation, et pendant une certaine durée, une alerte est déclenchée afin de solliciter l'intervention d'un proche ou d'une équipe médicale.

Malgré l'intérêt de cette solution, des changements de comportement peuvent survenir sans toutefois justifier un intervention. Comme évoqué plus haut, un changement d'heure ou de saison peut provoquer un changement de comportement sans pour autant qu'il soit nécessaire d'intervenir. Un tel système n'offre pas de solution technique satisfaisante pour prendre en compte de nouvelles conditions justifiant un changement dans les habitudes d'une personne suivie.

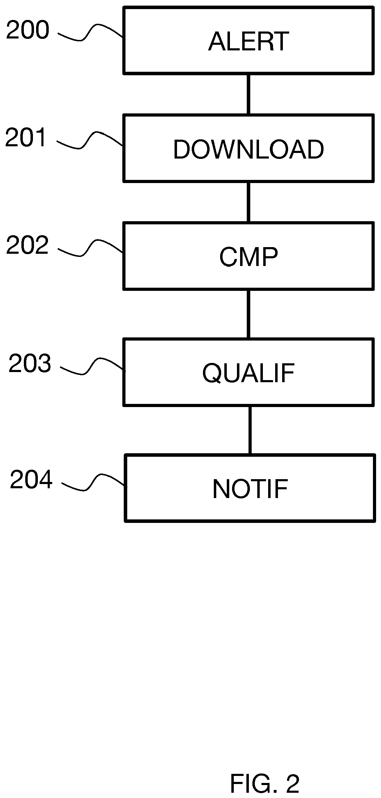

Nous allons maintenant décrire un procédé en référence conjointe à la figure 1 et à la figure 2, selon un mode particulier de réalisation.

La **figure 2** illustre les principales étapes d'un procédé de qualification d'une alerte non couvert par l'invention.

Le dispositif de contrôle de **104** de l'habitation 100 déclenche une alerte suite à l'observation d'une dérive importante de l'indice d'activité de la personne suivie à l'étape **200.** Cette alerte est interceptée par le serveur **112** pour être qualifiée avant de déclencher une intervention. Selon une réalisation particulière, le dispositif de contrôle **104** peut émettre une requête de qualification d'alerte à destination du serveur **112** avant de déclencher réellement l'alerte.

Lors d'une première étape **201,** le serveur **112** télécharge les indices d'activité quotidiens et les indices de référence établis pendant la période d'initialisation par les dispositifs de contrôles **104, 105, 106** et **107.** Selon une réalisation particulière, le serveur **112** télécharge la dérive de l'indice plutôt que l'indice quotidien et l'indice de référence. Le serveur de gestion **112** utilise pour cela le réseau de communication **113** qui le relie aux dispositifs de contrôle des habitations. De façon à ce que le serveur **112** connaisse les adresses réseau des différents dispositifs de contrôle, le procédé peut comporter une étape préalable de souscription au cours de laquelle les informations techniques concernant les systèmes de supervisions des 4 habitations sont communiquées au serveur. Par exemple, les dispositifs de contrôle peuvent être préconfigurés avec l'adresse du serveur **112** de façon à ce qu'ils puissent émettre un message d'enregistrement à la mise sous tension, le message d'enregistrement comportant un identifiant unique du système et une adresse de téléchargement.

Le serveur de gestion **112** effectue à l'étape **202** une comparaison entre la dérive de l'indice relevé pour l'habitation **100** à l'origine de l'alerte et une valeur représentative de la dérive de l'indice relevé pour au moins une autre des habitations **101, 102** et **103** enregistrées sur le serveur. Selon une réalisation particulière, la valeur représentative de la dérive de l'indice relevé pour les habitations **101, 102** et **103** est une moyenne de la dérive, un écart-type ou toute autre fonction statistique permettant d'obtenir une valeur représentative de cette dérive.

Selon une réalisation particulière, les dérives d'indices collectées à l'étape **201** sont pondérées selon la durée de mise en service des systèmes de supervision correspondants. Par exemple, si le système de supervision de l'habitation **101** a été mis en service avant le système de l'habitation **102,** un poids plus fort sera affecté à cette dérive lors du calcul de la dérive moyenne. Le procédé permet ainsi de privilégier les données issues de systèmes de supervision dont les données sont fiabilisées par une plus longue période d'observation.

À l'étape **203,** l'alerte est qualifiée par le serveur **112** selon le résultat de la comparaison. Par exemple, si la dérive relevée pour l'habitation **100** correspond à la moyenne des dérives constatées pour les autres habitations enregistrées sur le serveur **112,** l'alerte est qualifiée de fausse alerte. Si en revanche aucune corrélation ne peut être trouvée entre la dérive des indices de l'habitation 100 et des habitations **101, 102** et **103,** l'alerte est confirmée.

Lors d'une étape **204,** le serveur de gestion transmet une notification au dispositif de contrôle de l'habitation **100** comprenant la qualification de l'alerte. À la réception d'une telle requête, lorsque l'alerte est confirmée, le dispositif de contrôle déclenche une intervention. En revanche, si l'alerte est qualifiée de fausse alerte, le dispositif de contrôle ne déclenche pas d'intervention.

Ainsi, ce dispositif permet d'augmenter avantageusement la fiabilité du système de supervision en évitant de déclencher une intervention lorsque le changement de comportement reflète une tendance globale parmi les habitations supervisées.

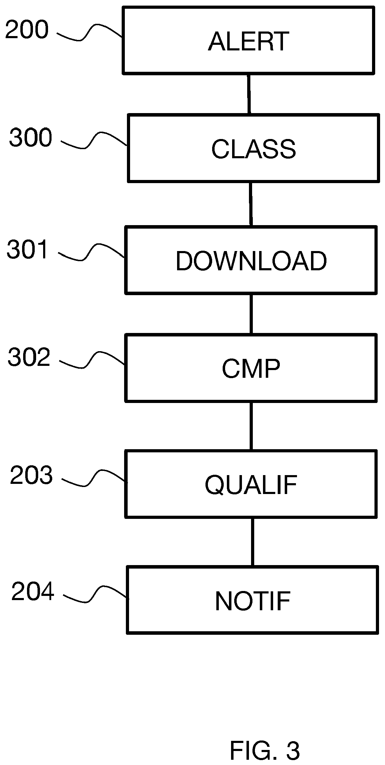

Un autre mode de réalisation particulier non couvert par l'invention va maintenant être décrit en référence à la **figure 3****.**

Selon une réalisation particulière, les informations transmises durant une phase d'enregistrement préalable des différents systèmes de supervision comprennent, outre les informations techniques permettant une bonne communication entre les dispositifs de contrôle et le serveur de gestion **112,** des informations de contexte domestique. Ces informations permettent de caractériser l'environnement supervisé et comprennent par exemple la configuration spatiale du logement, comme le nombre de pièces et d'étages, le fait qu'il s'agisse d'un logement individuel ou non, le nombre de salle de bain ou le fait qu'il existe ou non un jardin. Le contexte domestique peut également comprendre des informations relatives à la localisation géographique du logement et/ou des données physiologiques de la personne dont l'activité est supervisée, comme par exemple son poids, sa taille son sexe ou son âge. Ces données peuvent être mémorisées dans une base de données du serveur **112** par exemple.

Les étapes 200, 203 et 204 sont identiques à celles décrites en référence à la figure 2.

Lors d'une étape **300,** le serveur **112** identifie les systèmes de supervision dont le contexte domestique présente des similitudes avec le contexte domestique du système ayant émis une requête de qualification d'alerte. Par exemple, à l'interception d'une requête de qualification d'alerte émise pour l'habitation **100,** le serveur **112** consulte le contexte domestique du système de supervision de l'habitation et le compare avec les contextes domestiques des habitations **101, 102** et **103** afin de déterminer un ensemble d'habitations disposant d'un contexte similaire. Le serveur peut par exemple comparer le nombre de pièces des différents environnements supervisés afin constituer une classe de systèmes de supervision dont l'environnement est similaire. Dans cet exemple, le serveur **112** constitue une classe comportant les systèmes de supervision des habitations **101** et **102,** l'habitation **103** étant écartée car il dispose d'une pièce supplémentaire.

À l'étape **301,** seules les données relatives aux indices et dérive d'indices des habitations **100, 101** et **102** sont téléchargées.

Lors de l'étape **302,** le serveur **112** effectue une comparaison entre la dérive de l'indice constatée pour l'habitation **100** et la moyenne des dérives constatées pour les systèmes de supervision appartenant à la même classe que l'habitation **100.** Dans cet exemple, l'indice d'activité des habitations **101** et **102** est utilisé pour calculer une moyenne des dérives et effectuer une comparaison avec la dérive de l'indice d'activité de l'habitation **100.**

Le procédé permet ainsi de qualifier une alerte de façon particulièrement pertinente en confrontant la dérive d'un indice d'activité associé à un système de supervision particulier à la dérive moyenne de systèmes supervisant des environnements similaires.

Selon un mode de réalisation de l'invention, l'indice d'activité pour un système de supervision est établi à partir d'une pluralité d'indicateurs, et l'étape de qualification **203** comporte en outre des sous étapes d'identification de l'indicateur majoritaire ayant provoqué la dérive de l'indice du premier système, d'obtention de la dérive de l'indicateur correspondant pour le au moins un deuxième système de supervision, de comparaison de la dérive de l'indicateur majoritaire du premier système de supervision et d'une valeur représentative de la dérive de l'indicateur du au moins un deuxième système, et de mise à jour de la qualification de l'alerte en fonction du résultat de la comparaison.

Pour cela, le serveur **112** peut par exemple au moyen d'une requête de téléchargement spécifique, obtenir des données relatives aux dérives des indicateurs ayant servi à établir l'indice d'activité d'un système de supervision ayant sollicité la qualification d'une alerte, de façon à déterminer un indicateur dont la dérive est principalement responsable de la dérive de l'indice d'activité.

Par exemple, suite à la réception par le serveur **112** d'une requête de qualification d'alerte en provenance du dispositif de contrôle **104** de l'habitation **100,** celui-ci peut télécharger les différents indicateurs ayant servi à calculer l'indice d'activité. À partir de ces données, le serveur **112** peut déterminer l'indicateur ayant principalement contribué à la dérive de l'indice d'activité. Il peut s'agir par exemple d'un indicateur dont la dérive traduit une activité nocturne inhabituelle chez la personne suivie. Suite à l'identification de cet indicateur, le serveur **112** télécharge la dérive de cet indicateur pour les autres logements supervisés. Selon un mode particulier de réalisation, le serveur peut télécharger uniquement les dérives de cet indicateur pour les systèmes dont le contexte domestique est similaire à celui de l'habitation ayant déclenché l'alerte, c'est-à-dire les systèmes des habitations **101** et **102** dans cet exemple. Le serveur **112** peut alors calculer une valeur représentative de la dérive de cet indicateur pour les systèmes considérés, comme par exemple calculer une moyenne des dérives de l'indicateur, éventuellement pondérée selon la date de mise en service de l'installation. Le serveur **112** peut alors effectuer une comparaison entre la dérive de l'indicateur observée par le système ayant sollicité une qualification de l'alerte et la dérive moyenne de cet indicateur pour les autres systèmes considérés avant de qualifier l'alerte en fonction du résultat de la comparaison.

De cette façon, le procédé peut avantageusement confirmer une alerte détectée par un système de supervision alors que la comparaison seule de la dérive des indices d'activité aurait conduit à qualifier l'alerte de fausse alerte.

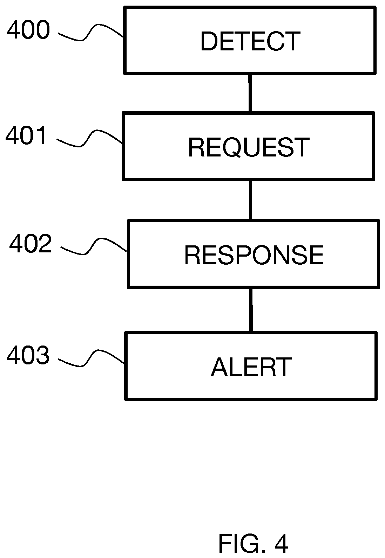

La **figure 4** illustre les principales étapes du procédé de demande de qualification d'alerte selon un mode de réalisation particulier. Lors d'une première étape **400,** une dérive sensible de l'indice d'activité est détectée par le système de supervision. Par exemple, l'indice s'être écarté de 10% de sa valeur de référence pendant une semaine. Afin de ne pas déclencher une intervention inappropriée, le système de supervision met en œuvre le procédé de demande de qualification d'alerte. Lors de l'étape **401,** une requête est émise vers un serveur de gestion, comme par exemple le serveur **112** de la figure 1. Cette requête comporte en particulier une information relative à la dérive de l'indice d'activité. Par exemple, la requête peut comporter la valeur de l'indice et la valeur de référence, ou bien la valeur de la dérive de l'indice. Selon une réalisation particulière, la requête peut également comporter une information relative à la dérive de l'indicateur principalement responsable de la dérive de l'indice.

Le serveur **112** reçoit cette requête à l'étape **200,** exécute les étapes du procédé de qualification d'alerte et émet une réponse à destination du système de supervision comportant la qualification de l'alerte.

Le système de supervision reçoit la réponse à la demande de qualification lors d'une étape **402.** Cette réponse comporte une information fonction d'une comparaison entre la dérive de l'indice d'activité du système de supervision et une valeur représentative de la dérive de l'indice d'activité d'au moins un deuxième système de supervision, cette information permettant au système de supervision de savoir si l'alerte est confirmée ou non. Lorsque l'alerte est confirmée, une alerte est émise à l'étape **403** à destination d'un serveur apte à déclencher une intervention au domicile de la personne. Dans le cas contraire, la dérive constatée est ignorée par le système de supervision.

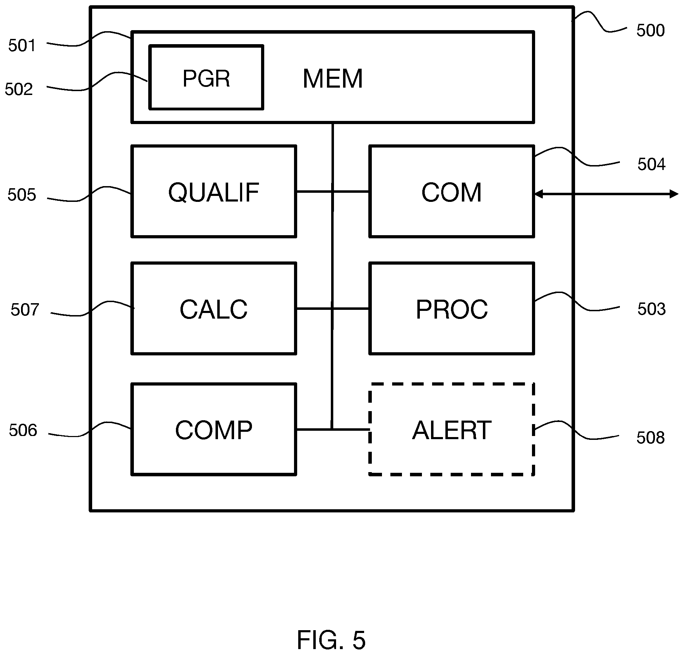

La **figure 5** illustre un dispositif **500** mettant en œuvre le procédé de qualification, selon un mode particulier de réalisation de l'invention. Le dispositif comprend un espace de stockage **501,** par exemple une mémoire MEM, une unité de traitement **503** équipée par exemple d'un processeur PROC. L'unité de traitement peut être pilotée par un programme **502,** par exemple un programme d'ordinateur PGR, mettant en œuvre le procédé de qualification d'alerte tel que décrit dans l'invention en référence aux **figures 1 à 3****,** et notamment les étapes d'obtention d'une dérive de l'indice d'activité du au moins un deuxième système de supervision, de comparaison de la dérive de l'indice d'activité du premier système de supervision et d'une valeur représentative de la dérive de l'indice d'activité du au moins un deuxième système, et de qualification de l'alerte en fonction du résultat de la comparaison.

À l'initialisation, les instructions du programme d'ordinateur **502** sont par exemple chargées dans une mémoire RAM (Random Access Memory en anglais) avant d'être exécutées par le processeur de l'unité de traitement **503.** Le processeur de l'unité de traitement **503** met en œuvre les étapes du procédé de composition selon les instructions du programme d'ordinateur **502.**

Pour cela, le dispositif comprend, outre la mémoire **501,** des moyens de communication **504** (COM) permettant au dispositif de se connecter à un réseau de télécommunication et d'échanger des données avec d'autres dispositifs par l'intermédiaire du réseau de télécommunications, et par exemple de recevoir une demande de qualification d'alerte, d'obtenir des informations relatives à la dérive d'un indice ou d'un indicateur et d'émettre un message de réponse lorsque l'alerte est qualifiée. Ces moyens de communication peuvent par exemple être une interface réseau. Le dispositif comprend également un calculateur **507** (CALC) apte à calculer une valeur représentative de la dérive de l'indice d'activité du au moins un deuxième système. Par exemple, le calculateur **507** peut calculer une dérive moyenne d'un indice pour un ensemble de systèmes de supervision. Selon un mode particulier de réalisation, le calculateur **507** peut appliquer une pondération aux différentes dérives avant d'en calculer une moyenne. Le dispositif comprend également un comparateur **506** apte à comparer une valeur de dérive d'un indice avec une dérive moyenne telle que calculée par l'unité **507.** Le dispositif comprend également un module de qualification, adapté pour qualifier une alerte à partir de résultat de la comparaison effectuée par l'unité **506.** De façon optionnelle, le dispositif peut également comporter une unité d'alerte **508** adapté pour solliciter une intervention au domicile supervisé dans le cas où une alerte est confirmée.

Selon un mode particulier de réalisation, le dispositif peut être intégré dans un serveur.

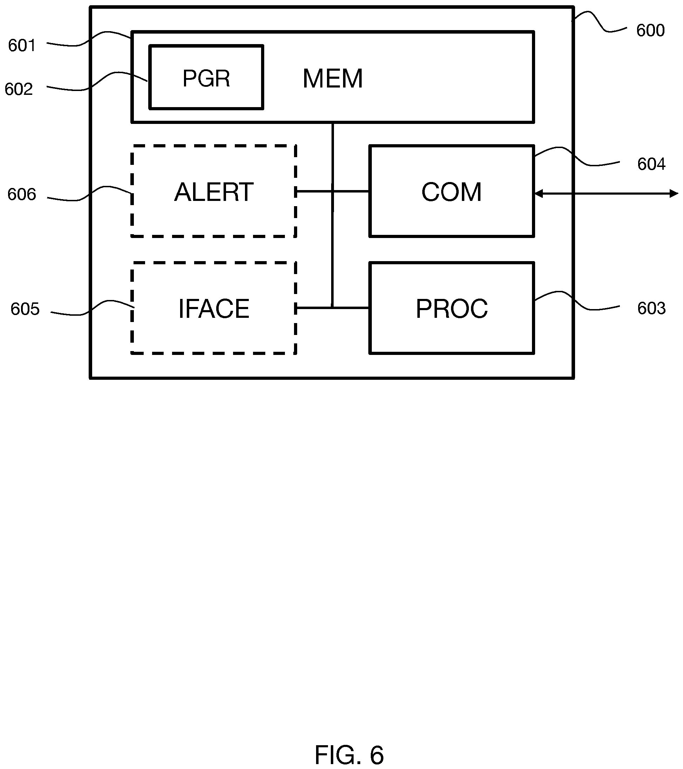

La **figure 6** illustre un dispositif **600** mettant en œuvre le procédé de demande qualification, selon un mode particulier de réalisation de l'invention. Le dispositif comprend un espace de stockage **601,** par exemple une mémoire MEM, une unité de traitement **603** équipée par exemple d'un processeur PROC. L'unité de traitement peut être pilotée par un programme **602,** par exemple un programme d'ordinateur PGR, mettant en œuvre le procédé de demande de qualification d'alerte tel que décrit dans l'invention en référence à la **figure 4****,** et notamment les étapes d'émission d'une requête de qualification d'alerte comportant une donnée relative à la dérive de l'indice d'activité, de réception d'un message comportant une qualification de l'alerte fonction d'une comparaison entre la dérive de l'indice d'activité du système de supervision et une valeur représentative de la dérive de l'indice d'activité d'au moins un deuxième système de supervision, et d'émission d'une alerte vers un serveur de traitement des alertes que lorsque qu'une alerte est confirmée.

À l'initialisation, les instructions du programme d'ordinateur **602** sont par exemple chargées dans une mémoire RAM (Random Access Memory en anglais) avant d'être exécutées par le processeur de l'unité de traitement **603.** Le processeur de l'unité de traitement **603** met en œuvre les étapes du procédé de demande de qualification selon les instructions du programme d'ordinateur **602.**

Pour cela, le dispositif comprend, outre la mémoire **601,** des moyens de communication **604** (COM) permettant au dispositif de se connecter à un réseau de télécommunication et d'échanger des données avec d'autres dispositifs par l'intermédiaire du réseau de télécommunications, et en particulier d'émettre une requête de demande de qualification d'alerte et de recevoir une réponse à cette requête comportant la qualification de l'alerte. Selon une réalisation particulière, le dispositif comprend en outre une unité d'interfaçage **605** avec un système de supervision. Cette unité d'interfaçage peut correspondre par exemple à une interface USB (Universal Serial Bus), Bluetooth, Ethernet ou encore par exemple à un bus de communication. Cette interface est adaptée pour communiquer avec le système de supervision selon un protocole d'échange de données. Le dispositif comprend également selon une réalisation particulière, une unité d'alerte **606,** adaptée pour transmettre une alerte confirmée à un serveur de traitement des alertes. L'unité d'alerte **606** est adaptée pour interpréter le message reçu en réponse à la demande de qualification et déclencher l'alerte lorsqu'elle est confirmée, par l'intermédiaire d'une interface réseau par exemple.

Selon un mode particulier de réalisation, le dispositif peut être intégrée dans un terminal, un système de supervision, ou encore une passerelle résidentielle.

## Revendications

1. Procédé de qualification d'une alerte parmi les alertes issues d'un premier système de supervision supervisant l'activité d'un individu mis en œuvre dans un serveur de gestion relié au premier système de supervision et à au moins un deuxième système de supervision d'un autre individu, le premier système étant adapté pour calculer un indice d'activité représentatif de l'activité de l'individu, des alertes étant déclenchées par le premier système de supervision lors d'une dérive de l'indice d'activité, une dérive correspondant à un écart de la valeur de l'indice par rapport à une valeur de référence, le procédé étant **caractérisé en ce que** l'indice d'activité pour un système de supervision est établi à partir d'une pluralité d'indicateurs et **en ce qu'**il comporte, à la réception (200) d'une alerte déclenchée par le premier système de supervision, les étapes suivantes :
- Obtention (201) d'une dérive de l'indice d'activité du au moins un deuxième système de supervision,
- Comparaison (202) de la dérive de l'indice d'activité du premier système de supervision et d'une valeur représentative de la dérive de l'indice d'activité du au moins un deuxième système,
- Qualification (203) de l'alerte en fonction du résultat de la comparaison,
- Identification de l'indicateur majoritaire ayant provoqué la dérive de l'indice du premier système,
- Obtention de la dérive de l'indicateur correspondant à l'indicateur majoritaire, pour le au moins un deuxième système de supervision,
- Comparaison de la dérive de l'indicateur majoritaire du premier système de supervision avec une valeur représentative de la dérive de l'indicateur correspondant du au moins un deuxième système, et
- Mise à jour de la qualification de l'alerte en fonction du résultat de la deuxième comparaison.

2. Procédé selon la revendication 1 **caractérisé en ce que** l'étape de deuxième comparaison est réalisée à partir de la dérive de l'indicateur majoritaire du premier système de supervision et d'une moyenne de la dérive de l'indicateur majoritaire de la pluralité de deuxième systèmes.

3. Procédé selon la revendication 1 ou 2 **caractérisé en ce que** les systèmes de supervision sont associés à des contextes domestiques et **en ce que** les systèmes de supervision sont regroupés en classes définies par au moins un critère de similitude des contextes domestiques auxquels ils sont associés, l'étape de première comparaison étant réalisée à partir de la dérive de l'indice d'activité du premier système de supervision et d'une moyenne de la dérive de l'indice d'activité des systèmes de supervision appartenant à la même classe que le premier système.

4. Procédé selon l'une quelconque des revendications précédentes **caractérisé en ce que** les dérives d'indice d'activité sont pondérées selon une date de mise en service du système de supervision correspondant.

5. Procédé selon l'une quelconque des revendications 3 à 4 **caractérisé en ce que** le au moins un critère de similitude entre les contextes domestiques comporte au moins un élément sélectionné parmi les éléments suivants :
- Une localisation géographique,
- La configuration spatiale d'un environnement, et
- Une caractéristique physiologique de l'individu.

6. Dispositif de qualification d'une alerte parmi les alertes issues d'un premier système de supervision supervisant l'activité d'un individu, le dispositif étant relié au premier système de supervision et à au moins un deuxième système de supervision d'un autre individu, le premier système étant adapté pour calculer un indice d'activité représentatif de l'activité de l'individu, des alertes étant déclenchées par le premier système de supervision lors d'une dérive de l'indice d'activité, une dérive correspondant à un écart de la valeur de l'indice par rapport à une valeur de référence, le dispositif étant **caractérisé en ce que** l'indice d'activité pour un système de supervision est établi à partir d'une pluralité d'indicateurs, et **en ce qu'**il comporte:
- Un module de communication (504) apte à recevoir une alerte déclenchée par le premier système de supervision,
- Un module de communication (504) apte à obtenir une dérive de l'indice d'activité du au moins un deuxième système de supervision,
- Un calculateur (507) apte à calculer une valeur représentative de la dérive de l'indice d'activité du au moins un deuxième système de supervision,
- Un comparateur (506) apte à comparer la dérive de l'indice d'activité du premier système de supervision avec la valeur représentative calculée,
- Un module d'identification de l'indicateur majoritaire ayant provoqué la dérive de l'indice du premier système,
- Un module de communication (504) apte à obtenir la dérive de l'indicateur correspondant à l'indicateur majoritaire, pour le au moins un deuxième système de supervision,
- Un comparateur (506) apte à comparer la dérive de l'indicateur majoritaire du premier système de supervision avec une valeur représentative de la dérive de l'indicateur correspondant du au moins un deuxième système, et
- Un module de qualification (505) apte à qualifier l'alerte en fonction du résultat de la
première comparaison et à mettre à jour la qualification de l'alerte en fonction du résultat de la deuxième comparaison.

7. Serveur de gestion **caractérisé en ce qu'**il comporte un dispositif selon la revendication 6.

8. Programme d'ordinateur comportant les instructions pour l'exécution du procédé de qualification selon l'une quelconque des revendications 1 à 5 lorsque le programme est exécuté par un processeur.

9. Support d'informations lisible par un processeur sur lequel est enregistré un programme d'ordinateur comprenant des instructions pour l'exécution des étapes du procédé de qualification selon l'une quelconque des revendications 1 à 5 .

## Patentansprüche

1. Verfahren zur Bewertung einer Warnmeldung innerhalb der Warnmeldungen, die von einem ersten Überwachungssystem, das die Aktivität einer Person überwacht, ausgegeben werden, umgesetzt in einem Verwaltungsserver, der mit dem ersten Überwachungssystem und mit mindestens einem zweiten Überwachungssystem einer anderen Person verbunden ist, wobei das erste System dazu angepasst ist, einen Aktivitätsindex zu berechnen, der für die Aktivität der Person repräsentativ ist, wobei im Fall einer Abweichung des Aktivitätsindexes Warnmeldungen durch das erste Überwachungssystem ausgelöst werden, wobei eine Abweichung einer Diskrepanz zwischen dem Wert des Indexes und einem Referenzwert entspricht, wobei das Verfahren **dadurch gekennzeichnet ist, dass** der Aktivitätsindex für ein Überwachungssystem basierend auf einer Vielzahl von Indikatoren erstellt wird und dass es beim Empfangen (200) einer Warnmeldung, die durch das erste Überwachungssystem ausgelöst wird, die folgenden Schritte umfasst:
- Erhalten (201) einer Abweichung des Aktivitätsindexes des mindestens einen zweiten Überwachungssystems,
- Vergleichen (202) der Abweichung des Aktivitätsindexes des ersten Überwachungssystems und eines Werts, der für die Abweichung des Aktivitätsindexes des mindestens einen zweiten Systems repräsentativ ist,
- Bewerten (203) der Warnmeldung in Abhängigkeit von dem Ergebnis des Vergleichs,
- Identifizieren des Hauptindikators, der die Abweichung des Indexes des ersten Systems herbeigeführt hat,
- Erhalten der Abweichung des Indikators, der dem Hauptindikator entspricht, für das mindestens eine zweite Überwachungssystem,
- Vergleichen der Abweichung des Hauptindikators des ersten Überwachungssystems mit einem Wert, der für die Abweichung des entsprechenden Indikators des mindestens einen zweiten Systems repräsentativ ist, und
- Aktualisieren der Bewertung der Warnmeldung in Abhängigkeit von dem Ergebnis des zweiten Vergleichs.

2. Verfahren nach Anspruch 1, **dadurch gekennzeichnet, dass** der zweite Vergleichsschritt basierend auf der Abweichung des Hauptindikators des ersten Überwachungssystems und einem Mittelwert der Abweichung des Hauptindikators der Vielzahl von zweiten Systemen durchgeführt wird.

3. Verfahren nach Anspruch 1 oder 2, **dadurch gekennzeichnet, dass** die Überwachungssysteme mit häuslichen Kontexten assoziiert sind und dass die Überwachungssysteme in Klassen eingeteilt sind, die durch mindestens ein Ähnlichkeitskriterium der häuslichen Kontexte, mit denen sie assoziiert sind, definiert sind, wobei der erste Vergleichsschritt basierend auf der Abweichung des Aktivitätsindexes des ersten Überwachungssystems und einem Mittelwert der Abweichung des Aktivitätsindexes der Überwachungssysteme, die zu derselben Klasse wie das erste System gehören, durchgeführt wird.

4. Verfahren nach einem beliebigen der vorhergehenden Ansprüche, **dadurch gekennzeichnet, dass** die Aktivitätsindexabweichungen gemäß einem Inbetriebnahmedatum des entsprechenden Überwachungssystems gewichtet werden.

5. Verfahren nach einem beliebigen der Ansprüche 3 bis 4, **dadurch gekennzeichnet, dass** das mindestens eine Ähnlichkeitskriterium zwischen den häuslichen Kontexten mindestens ein Element umfasst, das aus den folgenden Elementen ausgewählt ist:
- einem geografischen Standort,
- der räumlichen Konfiguration einer Umgebung und
- einer physiologischen Eigenschaft der Person.

6. Vorrichtung zur Bewertung einer Warnmeldung innerhalb der Warnmeldungen, die von einem ersten Überwachungssystem, das die Aktivität einer Person überwacht, ausgegeben werden, wobei die Vorrichtung mit dem ersten Überwachungssystem und mit mindestens einem zweiten Überwachungssystem einer anderen Person verbunden ist, wobei das erste System dazu angepasst ist, einen Aktivitätsindex zu berechnen, der für die Aktivität der Person repräsentativ ist, wobei im Fall einer Abweichung des Aktivitätsindexes Warnmeldungen durch das erste Überwachungssystem ausgelöst werden, wobei eine Abweichung einer Diskrepanz zwischen dem Wert des Indexes und einem Referenzwert entspricht, wobei die Vorrichtung **dadurch gekennzeichnet ist, dass** der Aktivitätsindex für ein Überwachungssystem basierend auf einer Vielzahl von Indikatoren erstellt wird und dass sie Folgendes umfasst:
- ein Kommunikationsmodul (504), das dazu fähig ist, eine Warnmeldung zu empfangen, die durch das erste Überwachungssystem ausgelöst wurde,
- ein Kommunikationsmodul (504), das dazu fähig ist, eine Abweichung des Aktivitätsindexes des mindestens einen zweiten Überwachungssystems zu erhalten,
- eine Recheneinheit (507), die dazu fähig ist, einen Wert, der für die Abweichung des Aktivitätsindexes des mindestens einen zweiten Überwachungssystems repräsentativ ist, zu berechnen,
- einen Komparator (506), der dazu fähig ist, die Abweichung des Aktivitätsindexes des ersten Überwachungssystems mit dem berechneten repräsentativen Wert zu vergleichen,
- ein Modul zum Identifizieren des Hauptindikators, der die Abweichung des Indexes des ersten Systems herbeigeführt hat,
- ein Kommunikationsmodul (504) das dazu fähig ist, die Abweichung des Indikators, der dem Hauptindikator entspricht, für das mindestens eine zweite Überwachungssystem zu erhalten,
- einen Komparator (506), der dazu fähig ist, die Abweichung des Hauptindikators des ersten Überwachungssystems mit einem Wert, der für die Abweichung des entsprechenden Indikators des mindestens einen zweiten Systems repräsentativ ist, zu vergleichen, und
- ein Bewertungsmodul (505), das dazu fähig ist, die Warnmeldung in Abhängigkeit von dem Ergebnis des ersten Vergleichs zu bewerten und die Bewertung der Warnmeldung in Abhängigkeit von dem Ergebnis des zweiten Vergleichs zu aktualisieren.

7. Verwaltungsserver, **dadurch gekennzeichnet, dass** er eine Vorrichtung nach Anspruch 6 umfasst.

8. Computerprogramm, das die Anweisungen für die Ausführung des Bewertungsverfahrens nach einem beliebigen der Ansprüche 1 bis 5 umfasst, wenn das Programm von einem Prozessor ausgeführt wird.

9. Von einem Prozessor lesbarer Informationsträger, auf dem ein Computerprogramm aufgezeichnet ist, das Anweisungen für die Ausführung der Schritte des Bewertungsverfahrens nach einem beliebigen der Ansprüche 1 bis 5 beinhaltet.

## Claims

1. Method for qualifying an alarm among the alarms stemming from a first supervision system supervising the activity of an individual, which method is implemented in a management server connected to the first supervision system and to at least one second supervision system of another individual, the first system being adapted to compute an activity index that is representative of the activity of the individual, alarms being triggered by the first supervision system in the event of a drift in the activity index, a drift corresponding to a difference in the value of the index in relation to a reference value, the method being **characterized in that** the activity index for a supervision system is established on the basis of a plurality of indicators and **in that** it has, on reception (200) of an alarm triggered by the first supervision system, the following steps:
- obtainment (201) of a drift in the activity index of the at least one second supervision system,
- comparison (202) of the drift in the activity index of the first supervision system and a value that is representative of the drift in the activity index of the at least one second system,
- qualification (203) of the alarm according to the result of the comparison,
- identification of the preponderant indicator that has caused the drift in the index of the first system,
- obtainment of the drift in the indicator corresponding to the preponderant indicator, for the at least one second supervision system,
- comparison of the drift in the preponderant indicator of the first supervision system with a value that is representative of the drift in the corresponding indicator of the at least one second system, and
- update of the qualification of the alarm according to the result of the second comparison.

2. Method according to Claim 1, **characterized in that** the step of second comparison is performed on the basis of the drift in the preponderant indicator of the first supervision system and an average for the drift in the preponderant indicator of the plurality of second systems.

3. Method according to Claim 1 or 2, **characterized in that** the supervision systems are associated with domestic contexts and **in that** the supervision systems are grouped into classes that are defined by at least one criterion of similarity for the domestic contexts with which they are associated, the step of first comparison being performed on the basis of the drift in the activity index of the first supervision system and an average for the drift in the activity index of the supervision systems belonging to the same class as the first system.

4. Method according to any one of the preceding claims, **characterized in that** the activity index drifts are weighted according to a startup date for the corresponding supervision system.

5. Method according to either one of Claims 3 and 4, **characterized in that** the at least one criterion of similarity between the domestic contexts has at least one element selected from among the following elements:
- a geographical location,
- the spatial configuration of an environment, and
- a physiological characteristic of the individual.

6. Device for qualifying an alarm among the alarms stemming from a first supervision system supervising the activity of an individual, the device being connected to the first supervision system and to at least one second supervision system of another individual, the first system being adapted to compute an activity index that is representative of the activity of the individual, alarms being triggered by the first supervision system in the event of a drift in the activity index, a drift corresponding to a difference in the value of the index in relation to a reference value, the device being **characterized in that** the activity index for a supervision system is established on the basis of a plurality of indicators, and **in that** it has:
- a communication module (504) that is capable of receiving an alarm triggered by the first supervision system,
- a communication module (504) that is capable of obtaining a drift in the activity index of the at least one second supervision system,
- a computer (507) that is capable of computing a value that is representative of the drift in the activity index of the at least one second supervision system,
- a comparator (506) that is capable of comparing the drift in the activity index of the first supervision system with the computed representative value,
- an identification module for identifying the preponderant indicator that has caused the drift in the index of the first system,
- a communication module (504) that is capable of obtaining the drift in the indicator corresponding to the preponderant indicator, for the at least one second supervision system,
- a comparator (506) that is capable of comparing the drift in the preponderant indicator of the first supervision system with a value that is representative of the drift in the corresponding indicator of the at least one second system, and
- a qualification module (505) that is capable of qualifying the alarm according to the result of the first comparison and of updating the qualification of the alarm according to the result of the second comparison.

7. Management server **characterized in that** it has a device according to Claim 6.

8. Computer program having the instructions for executing the qualification method according to any one of Claims 1 to 5 when the program is executed by a processor.

9. Information storage medium that can be read by a processor on which is recorded a computer program comprising instructions for executing the steps of the qualification method according to any one of Claims 1 to 5.
